# EUROPEAN PATENT APPLICATION

(11) **EP 2 182 068 A1**
(43) Date of publication of application: **05.05.2010**
(21) Application number: 08167775.9
(22) Date of filing: 28.10.2008
(51) Int. Cl.: C12N 15/62, C12N 15/70, A61K 39/02

(54) **Use of bacteria for the sensing and killing of cancer cells**

(71) Applicant: DKFZ Deutsches Krebsforschungszentrum, 69120 Heidelberg (DE)
(72) Inventor: The designation of the inventor has not yet been filed
(74) Representative: Dick, Alexander

(57) **Abstract**

The present invention relates to the field of cancer therapy. Specifically, a chemotaxis fusion-receptor that directs bacteria towards tumors is disclosed. Further encompassed by the present invention is a bacterial cell that comprises at least one chemotaxis fusion-receptor and, preferably, a killing module for the destruction of tumor cells.

## Description

The present invention relates to the field of cancer therapy. Specifically, a chemotaxis fusion-receptor that directs bacteria towards tumors is disclosed. Further encompassed by the present invention is a bacterial cell that comprises at least one chemotaxis fusion-receptor and, preferably, a killing module for the destruction of tumor cells.

Cancer constitutes the fourth leading cause of death in Western countries. As the average age in the Western population steadily rises, so do cancer-related deaths indicating that cancer will be one of the most common causes of death in the 21^{st} century. The aggressive cancer cell phenotype is the result of a variety of genetic and epigenetic alterations leading to deregulation of intracellular signaling pathways. This leads to uncontrolled proliferation of the affected cells and, thus, to the formation of a tumor. Malignant tumors have three characteristics: (i) they infiltrate healthy tissues, (ii) they destroy the surrounding tissue, and (iii) they form metastases in other parts of the body.

As long as the cancer has not formed metastases, it can be treated by surgery or radiotherapy. Both methods, however, are not suited for the systemic treatment of cancer that has already formed metastases. In most of these cases chemotherapy is the only available option for systemic cancer treatment. Traditional chemotherapeutic agents are cytotoxic substances that target preferably rapidly dividing cells. Unfortunately cancer cells are not the only cells which display high proliferation rates. Many cells of the body, such as cells of the immune system, have high proliferation rates, too. Thus, classical chemotherapy usually causes severe side effects that sometimes necessitate the cessation of treatment. Furthermore, cancer cells are often resistant against one or more of the applied pharmaceuticals or develop resistances during the course of treatment.

In recent years systemic cancer treatments have become available which target cancer cells with higher specificity. Imatinib, for example, targets cancer cells that are characterized by an abnormal tyrosine kinase, which is formed by the fusion of the bcr and abl gene. Because this fusion gene is only present in cancer cells, normal cells are considerably less affected by this treatment as compared to traditional chemotherapy. However, Imatinib can only be used for the treatment of cancers with the bcr-abl fusion gene. Thus, a higher specificity of the novel cancer treatments often means that their efficacy is limited to a restricted number of cancers. For this reason, there is a continuing need for the development of novel cancer therapies that enable the treatment of cancer more generally.

The development of safe and effective cancer therapies has been hampered by the difficulties in targeting cancer cells (i) specifically and (ii) completely. One of the reasons for these deficiencies is the fact that non-living substances generally do not actively accumulate close to the cancer cells. As the vascular system in tumor tissue is often poorly developed, blood flow in tumors is often disorganized and variable. This creates heterogenous microenvironments within the tumor, because passive spreading by diffusion is very inefficient. Medicaments often do not reach poorly supplied regions of the tumor. Bacteria, in contrast, have mechanisms to actively accumulate in tumor tissues. The main mechanisms are specific chemotaxis, preferred growth in tumor tissue and hypoxic germination. Facultative anaerobes from the genera *Salmonella* and *Escherichia* have been shown to accumulate in tumors by chemotaxis. Due to this specific accumulation in tumors it has been tried to use bacteria for cancer therapy. In order to make bacteria toxic towards cancer cells, several methods have been used. Bacteria have been engineered to produce bioactive molecules or to transfer the genes encoding such molecules into cancer cells. Furthermore, bacteria have been equipped with genes encoding for pro-drug activating enzymes. For a review of bacterial cancer treatments see St Jean AT, 2008, Curr. Opin. Biotechnol. 19: 511-517.

Bacteria are attracted to certain substances ("attractants"), such as carbon sources or amino acids, while they avoid other substances ("repellents"), e.g. weak organic acids. This phenomenon is known as bacterial chemotaxis. Normally, bacteria swim for an average period of 1 second in a straight line, then change their direction ("tumble") in a random fashion and swim again in a straight line. This is called "random walk". When moving in a concentration gradient of an attractant towards the higher concentration, the interval between the tumbling movements increases so that the movement along the gradient is stabilized. Since bacteria are too small to sense a gradient along their cells, the spatial signal of a concentration gradient is intracellularly transformed into a temporal signal. The key components for the sensing of concentration gradients are methyl-accepting chemotaxis proteins (MCPs). The periplasmic domain of these proteins contains several methylation sites. An increasing methylation of the MCP leads to an increasing duration of the intervals between the tumbling movements. The methylation state of the MCP is determined by the interplay of two enzymes, a permanently active methyltransferase and a methylase. The binding of an attractant to the receptor leads to a lower affinity of the methylase for the MCP so that the balance of methylation and demethylation is shifted towards methylation. Conversely, the binding of a repellent to a suitable receptor indirectly increases the activity of the methylase so that the MCP is demethylated. This favours tumbling movements of the bacterial cell. Different MCPs act on the same target proteins, i.e. CheA and CheY. Thus, the signals different chemotaxis receptors can be integrated to produce a response of the bacterial cell. For the structure and function of chemotaxis receptors see Mowbray and Sandgren (1998) Structural Biology 124: 257-275.

The previous attempts to use bacteria in cancer treatments have focussed on means to increase the ability of the bacteria to kill cancer cells. Regarding the delivery of the bacteria to the cancer cells the state of the art relies on the innate capabilities of the bacterial strains used in the experiments. This severely limits the possibilities of this therapeutical approach. Those bacteria that accumulate best in tumors are not necessarily also the best candidates for killing of cancer cells. The ability to equip almost any bacterial strain with the ability to accumulate close to cancer cells is clearly desirable. In addition to this, as long as the naturally occuring chemotaxis systems of bacteria are used, the specificity and sensitivity of these systems cannot be influenced.

The technical problem is solved by the embodiments characterized in the claims and below.

The present invention relates to a chemotaxis fusion-receptor comprising
a) a signal transduction component; and
b) a periplasmic domain comprising a ligand binding site for a ligand selected from the ligands given in table 1.

A "chemotaxis fusion-receptor" as referred to in the present application is a methyl-accepting chemotaxis protein that comprises two components which originate from at least two different proteins. The signal transduction component comprises two transmembrane domains and the cytoplasmic domain. These domains originate, preferably, from a bacterial chemotaxis receptor. They are functionally linked to a periplasmic domain comprising a ligand binding site, so that the binding of a ligand activates the cytoplasmatic domain. The periplasmic domain is located between the two transmembrane domains. The ligand binding site originates from a different protein, preferably, from a receptor, i.e. an antibody or protein listed in table 1.

The cytoplasmic domains at the N-termini of naturally occurring methyl-accepting chemotaxis proteins (MCPs) are highly conserved among those proteins. The ligand binding sites in the periplasmic domains, in contrast, are highly variable. Thus, MCPs couple various different signal inputs to the same output, i.e. a positive or negative chemotaxis. As the signal transduction of an MCP functions equally well with different ligand binding sites in the periplasmic domain, it is possible to replace the naturally occurring binding site of an MCP by a selected ligand binding site from another protein. Such a chemotaxis fusion-receptor enables the construction of bacterial cells which display positive or negative chemotaxis for a ligand which is not recognized by naturally occurring bacterial chemotaxis receptors. Bacterial chemotaxis systems with customized specificities can be found in the examples.

In a preferred embodiment of the present invention the chemotaxis fusion-receptor comprises elements from the chemotaxis receptor Tar of Escherichia coli and the LuxQ-protein of Vibrio harveyi.

The aspartate receptor Tar (SEQ iD NO. 1) is a 60 kDa protein with about 2500 copies per cell. The smallest units of the receptor are dimers, but the major species in the membrane are tetramers. There is no evidence that tar can form heterodimers with other MCP (methyl-accepting chemotaxis protein). The receptor has a very high helical content of about 80 %. The following description of its structure is that of the tar receptor from *E. coli.* The N-terminal cytoplasmic segment is very small (residues 1-6) and can be altered greatly without effecting the function very much. The periplasmic region (residues 31-188) is responsible for ligand binding. In this part the sequence is very low conserved, because of the need of binding different chemoeffectors. In the absence of a ligand the periplasmic part is a symmetric dimer, where each subunit is built up by an antiparallel four-helix bundle. The tar receptor has two transmembrane regions flanking the periplamsic domain: residues 7-30 (TM1) and residues 189-212 (TM2). Both transmembrane segments have a clear helix pattern. The cytoplasmic region, responsible for signal transduction, has a size of about 37 kDa including residues 213-553 of the protein. It is the most conserved part, with sequence identity of -70 % between Tar and Tsr. Also it is the least understood domain. Residues 213-259 are referred to as the linker region and its integrity is crucially important for receptor function (Mowbray and Sandgren (1998) Journal of Structural Biology, 124: 257-275). LuxQ (SEQ iD NO. 2) is a transmembrane receptor with similar structure to that of Tar. It is part of the quorum-sensing system of *Vibrio harveyi.* LuxQ binds the complex of LuxP and Autoinducer-2 (AI-2). AI-2 is produced by LuxS, 159 amino acids long and with a molecular weight of 17.6 kDa. In the natural quorum-sensing system binding of AI-2 results in gene regulation. LuxP is 365 amino acids in length and has a molecular weight of 41-kDa. Its signal sequence is proteolytically removed upon translocation into the periplasm, yielding mature LuxP. LuxQ is 594 amino acids long with a molecular weight of 67 kDa. It has, similar to Tar, two transmembrane Domains flanking the periplasmic domain (residues 39-280). The cytoplasmic domain (residues 299-859) is responsible for signal transduction, and not important for the chemotaxis fusion receptor. The N-terminal sequence of the chemotaxis fusion-receptor comprises the amino acid residues 1 to 298 of SEQ ID. NO. 2, i.e. transmembrane domain 1, the periplasmic domain and transmembrane domain 2 of LuxQ. The periplasmic domain is, preferably, exchanged completely or partially against the ligand binding domain of a receptor able to bind a ligand. At the carboxy-terminus the sequence of the chemotaxis fusion-receptor comprises the amino acid residues 225 to 564 of Seq ID NO. 1, i.e. the cytoplasmic domain of the chemotaxis-receptor Tar of Escherichia coli.

In another preferred embodiment of the present invention the N-terminal sequence of the chemotaxisfusion receptor comprises the amino acid residues 1 to 280 of SEQ ID. NO. 2, i.e transmembrane domain 1 and the periplasmic domain of LuxQ. The periplasmic domain is, preferably, exchanged completely or partially against the ligand binding domain of a receptor able to bind a ligand. At the carboxy-terminus the sequence of the chemotaxis fusion-receptor comprises the amino acid residues 200 to 564 of Seq ID NO. 1, i.e. transmembrane domain 2 and the cytoplasmic domain of Tar.

In another preferred embodiment of the present invention the first transmembrane domain and the periplasmic domain of the chemotaxis fusion-receptor are exchanged for the ligand binding domain of a receptor able to bind a ligand. Also preferably, said exchange also includes the second transmembrane domain. This embodiment is preferred for receptors able to bind a ligand which are structurally similar to Tat or LuxQ, i.e. comprise at the N-terminus a first transmembrane domain, a periplasmic domain and a second transmembrane domain or at least a periplasmic domain and a transmembrane domain.

Suitable ligands for the binding site are chemical compounds, preferably proteins or small molecules, that are secreted by cancer cells. Preferred ligands are secreted either exclusively by cancer cells or are secreted by the cancer cells in an increased or decreased amount compared to a corresponding non-cancer cell (normal cell). More preferred are ligands that are secreted by cancer cells in higher amounts than by normal cells. Even more preferred are ligands that are exclusively secreted by cancer cells. The most preferred ligands are those given in table 1.

Preferred receptors able to bind a ligand are given in table 1.

Advantageously, the chemotaxis fusion-receptor of the present invention enables a flexible use of bacterial host cells in cancer therapy. A broad range of bacterial host cells capable of chemotaxis can be equipped with the chemotaxis fusion-receptor of the present invention by well established genetic methods. Furthermore, it is possible to select the ligand of the chemotaxis system at will. This enables the construction of bacteria that recognize specific types of cancer cells only depending on the chosen ligand. While the currently used bacteria are only reported to target solid tumors, the present invention enables the construction of chemotaxis fusion-receptors for the targeting of leukaemia. The combination of more than one chemotaxis fusion-receptor enables the integration of signals from more than one ligand thus further increasing the flexibility of the system.

The present invention further relates to a polynucleotide comprising at least one nucleic acid encoding the chemotaxis fusion-receptor of the present invention.

The polynucleotide of the present invention shall be provided, preferably, either as an isolated polynucleotide (i.e. isolated from its natural context) or in genetically modified form. The polynucleotide, preferably, is DNA including cDNA or RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

Moreover, the present invention relates to a vector comprising the polynucleotide of the present invention.

The term "vector", preferably, encompasses phage, plasmid or vectors as well artificial chromosomes, such as bacterial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

More preferably, the vector of the invention the polynucleotide is operatively linked to expression control sequences allowing expression in prokaryotic cells or isolated fractions thereof. Expression of said polynucleotide comprises transcription of the polynucleotide, preferably into a translatable mRNA. Possible regulatory elements permitting expression in prokaryotic host cells comprise, e.g., the *lac*, *trp* or *tac* promoter in *E. coli.* Moreover, inducible expression control sequences may be used in an expression vector encompassed by the present invention. Such inducible vectors may comprise *tet* or *lac* operator sequences or sequences inducible by heat shock or other environmental factors. Suitable expression control sequences are well known in the art. Beside elements which are responsible for the initiation of transcription such regulatory elements may also comprise transcription termination signals. In this context, suitable expression vectors are known in the art. Preferred expression vectors are given in Seleem et al. (2008), Gene 421: 95-98 and Shkoporov et al. (2008) Biotechnol. Lett. 30: 1983-1988. Preferably, said vector is an expression vector and a gene transfer or targeting vector. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The present invention further relates to a bacterial host cell comprising the chemotaxis fusion-receptor of the present invention, the polynucleotide of the present invention, or the vector of the present invention.

A "bacterial host cell" as referred to in the present application is, preferably, a bacterial cell which is capable of surviving in an animal organism without causing systemic infection. To limit the pathogenic potential of such a bacterial host cell it is envisaged to limit its ability to proliferate in the animal body, e.g. by deleting genes that are required for iron uptake. A further criterion for the suitability of a bacterial host cell according to the invention is the possibility to functionally integrate the above described chemotaxis fusion-receptor so that the chemotaxis fusion-receptor can elicit positive or negative chemotaxis upon binding of the ligand.. Preferred bacteria to be used as host cells according to the present invention belong to the genera *Clostridium*, *Bifidobacterium*, *Salmonella* and *Escherichia.* Especially preferred are *S. thyphimurium*, *E. coli, C. novyi* and *C. acetobutylicum.*

The present invention further relates to a bacterial host cell comprising at least two chemotaxis fusion-receptors. In many cases cancer cells can not be identified by the secretion of unique compounds, i.e. compounds that are only secreted by cancer cells and not by normal cells. Thus, it is not possible to use a single compound as a target for bacterial chemotaxis in order to direct the bacterial host cells to the cancer cells. However, compounds that are secreted by many or even all cell types of the animal body may be secreted by cancer cells in different amounts. Hence, cancer cells show different secretion profiles of these compounds as compared to normal cells. As described in the introduction it is possible to integrate the signals from different compounds. The combination of at least two chemotaxis fusion-receptors specific for different ligands in one bacterial host cell increases its specificity for cancer cells, because it enables the differentiation between cells with quantitatively but not qualitatively different secretion profiles.

Preferably, the expression level of a receptor in a bacterial host cell can be used to achieve a different sensitivity of the cell for the respective ligand. For example, a bacterial host cell expressing high amounts of a chemotaxis fusion-receptor according to the present invention is capable of responding to lower levels of the ligand than a bacterial host cell expressing a lower amount of the chemotaxis fusion-receptor of the present invention. Also preferably, the affinity of the receptor for the ligand is changed by directed or undirected evolution. Further preferred, is the addition of specificity to the sensing by combining receptors for attracting ligands (i.e. ligands which elicit positive chemotaxis) and repelling ligands (i.e. ligands which elicit negative chemotaxis) in the same bacterial host cell.

The term "cancer" as referred to in the present application refers to cancer of haematopoietic origin or solid cancers. Preferably it refers to leukaemia, lymphoma, breast cancer, gastric cancers, colon cancer, lung cancer, cancers of the skin, brain tumors, cancers of the oesophagus, kidney cancer, liver cancer, osteosarcoma, prostrate cancer, cervix carcinoma. Most preferably the term "cancer" refers to melanoma, lung cancer and oral submucous fibrosis.

Furthermore, the present invention, preferably, relates to a bacterial host cell comprising at least one chemotaxis fusion-receptor and at least one gene which enables the bacterial host cell to kill cancer cells. Said bacterial host cell, preferably, kills the cancer cell by inducing apoptosis or necrosis.

In a preferred embodiment of the present invention the killing module comprises a gene which encodes at least one physiologically active molecule to induce a physiological response in the cancer cells. Preferred physiologically active molecules that induce biological responses in cancer cells are TNFα, interleukin-2, molecules inhibiting hypoxia inducible factor 1α, endostatin, and thrombospondin 1. More preferred is a killing module which enables the bacterial host cell to produce a bacteriocin. The anti-cancer properties of bacteriocins are discussed by Comut et al. (2008) Am. J. Oncol. 31: 399-404. Most preferred is a killing module which enables the bacterial host cell to produce a colicin. Colicins are peptides produced by the enterobacterium *Escherichia coli.* They are lethal for other bacteria. Interestingly, colicins are toxic for cancer cells as well (Smarda J et al., 2001 Folia Microbiol. (Praha) 47: 11-13). To be effective the colicins have to be taken up by the target cell. Most colicins have either nuclease activity or form pores in the bacterial cell membrane. The nuclease activity degrades the genome of the target cell. Colicins that form pores in the cell membrane make it permeable for inorganic ions and, thus, destroy the electrochemical gradient across the membrane. The target cell dies because ATP-production stops. Colicins are potentially lethal for the bacterial host cell, too. Thus, the killing module preferably comprises additionally an immunity gene against the colicin under the control of a constitutive promoter. Furthermore, the killing module comprises, preferably, a lysis gene that induces lysis of the bacterial host cell to liberate the produced colicins. Especially preferred killing modules comprise the colicins E9, a colicin with nuclease activity, or the colicin E1, a pore-forming colicin.

It is further envisaged to equip the bacterial host with a killing module that enables the transfer of genes encoding physiologically active proteins into the cancer cells. This process is known as bactofection (Palffy R et al., 2006, Gene Ther. 13: 101-105).

Furthermore, a preferred killing module in the context of the present invention comprises at least one gene encoding at least one pro-drug activating enzyme. If the expression of such an enzyme occurs preferably close to the cancer cells, the activation of the pro-drug happens at a higher rate close to the cancer cells so that the effective concentration of the active drug is high at the cancer cells and comparatively low at other places. Thus, a maximal therapeutic effect on the cancer cells is combined with little undesired side effects of the drug. A preferred pro-drug activating enzyme is cytosine deaminase which converts the pro-drug 5-fluorocytosine into the chemotherapeutic 5-fluorouracil.

All of the above described killing modules are, preferably, under the control of promoters that ensure that the killing module is only activated after the bacteria have reached the cancer cells. Thus, in a preferred embodiment of the present invention the killing module is controlled by a promoter which is specifically activated in the tumor-environment. A preferred promoter is the promoter controlling the formiate dehydrogenase gene in *E. coli.* It is active under hypoxic conditions. Such conditions are frequently encountered in tumor tissue.

In another preferred embodiment of the present invention the promoter is P_{LuxR} from *Vibrio harveyi.* In this case the bacterial host cell comprises a further gene encoding a modified LuxR-protein. In the modified LuxR-protein the ligand binding domain of the original LuxR-protein is exchanged for a binding domain recognizing a compound produced by a tumor cell. Preferred binding domains are derived from the antibodies, receptors and proteins given in table 1, third column. However, since the LuxR-protein is located intracellularly, a modified LuxR-protein can only be used, if the chosen ligand is able to permeate the bacterial cell membrane.

In yet another preferred embodiment of the present invention the promoter controlling the expression of the killing module is controlled by an external stimulus. Preferred promoters that are controlled by external stimuli react to non-toxic small molecules or radiation. Especially preferred are the P_{BAD}-promoter of E. coli which is induced by L-arabinose or the radiation-induced recA-promoter sequence. Furthermore, the use of a system comprising the rtTA-protein or the tTA-protein in combination with the tetO-operator is envisaged by the present invention for the expression control of the killing module.

In another preferred embodiment of the present invention any of the above described bacterial host cells is used for the manufacture of a pharmaceutical composition for treating cancer in patient.

The term "pharmaceutical composition" as used in the present patent application comprises the bacterial host cell of the present invention and, preferably, one or more pharmaceutically acceptable carrier(s). The pharmaceutical compositions are, preferably, administered systemically. Preferred routes of administration are intravenous or parenteral administration.

Moreover, the bacterial host cells can be administered in combination with other drugs either in a common pharmaceutical composition or as separated pharmaceutical compositions wherein said separated pharmaceutical compositions may be provided in form of a kit of parts.

The bacterial host cells are, preferably, administered in conventional dosage forms prepared by combining the drugs with standard pharmaceutical carriers according to conventional procedures. These procedures may involve mixing, suspending or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example a liquid. Exemplary of liquid carriers are phosphate buffered saline solution, syrup, oil, water, emulsions, various types of wetting agents and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the bacterial host cell. Examples of such diluents are physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days. Dosage recommendations shall be indicated in the prescribers or users instructions in order to anticipate dose adjustments depending on the considered recipient.

The term "treating" refers to ameliorating the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

A "subject" as referred to herein is, preferably, an animal. More preferably, it is a mammal. Even more preferably it is a cat, dog, mouse, rat, guinea pig, pig, horse or sheep. Most preferably, the patient is a human. Preferably, the patient to be treated with one of the pharmaceutical compositions described above is suffering from cancer or suspected to suffer from cancer.

The present invention, finally, relates to a method for treating cancer in a subject comprising the step of administering any of the above described bacterial host cells or compositions to a subject suffering from cancer in a therapeutically active amount.

All the references cited in this specification are herewith incorporated by reference with respect to their entire disclosure content and the disclosure content specifically mentioned in the specification.

**Table 1: Preferred ligands and antibodies, receptors and proteins capable of binding the ligands**

| **Ligands** | **Tumor** | **Receptors able to bind the ligand (Antibody/Receptor/Protein)** | **Reference** |
|---|---|---|---|
| cripto-1 | Melanoma | ALK4 | - Strizzi 2008. Landesbioscience, Vol. 7:13, p. 1932 |
| | | | - Bianco 2002, Mol. Cell. Biol., Vol .274:13, p. 8624 |
| Nodal | Melanoma | ALK4, ALK7 | - Topczewska 2006. nature medicine, Vol. 12:8, pp. 925-927 |
| | | | - Reissmann 2001, Genes & Devel., Vol. 15, p. 2010 f. |
| DOPA | Melanoma | OA-1 | - Slominski 2001, Arch Pathol Lab Med, Vol. 125, p. 1301 |
| | | | - Lopez 2008, Plos Biology, Vol. 6:9, p. 1861 |
| 5-S-cysteinyldopa (5-SCD) | Melanoma | OA-1 | - Hartleb 2001, J Chrom B, Vol. 764, p. 409 |
| | | | - Lopez 2008, Plos Biology, Vol. 6:9, p. 1861 |
| 6-hydroxy-5-methoxyindole-2-carboxylic acid (HMI2C) | Melanoma | Anti-HMI2C Antibody | - Hartleb 2001, J. Chrom. B, Vol. 764, p. 409 |
| | | | - Kammeyer 1992, J Immun Meth, Vol. 156, p. 61 |
| YKL-40 | Solid tumors | YKL-40 receptor | Johansen 2006, Cancer Epidemiology, Biomarkers & Prevention, Vol. 15:2, pp. 194, 196 |
| Secreted frizzled-related protein 4 | Oral Submucous Fibrosis | Wg (wingless) | - Li 2008, Cancer Epidemiology, Biomarkers & Prevention, Vol. 17:9, p. 2252 |
| | | | - Üeren 2000, J. Biol. Chem., Vol. 275:9, p. 4374 |
| Loricrin | Oral Submucous Fibrosis | Loricrin-C13 Antibody | - Li 2008, Cancer Epidemiology, Biomarkers & Prevention, Vol. 17:9, pp. 2249 ff. |
| | | | - Santa Cruz Biotechnology INC http://datasheets.scbt.com/sc-51130.pdf |
| Thrombospondin | Oral Submucous Fibrosis | Integrin-associated Protein (CD47) | - Li 2008, Cancer Epidemiology, Biomarkers & Prevention, Vol. 17:9, p. 2252 |
| | | | - Frazier 1999, J Biol Chem, Vol. 274:13, p. 8554 f. |
| Sarcolipin | Oral Submucous Fibrosis | Sarcoplasmic reticulum Ca2+-ATPase - (SERCA1) | Li 2008, Cancer Epidemiology, Biomarkers & Prevention, Vol. 17:9, p. 2252 |
| | | | - Odermatt 1998, J Biol. Chem, Vol 273:20, p. 12360 ff. |
| Corneodesmosin | Oral Submucous Fibrosis | Monoclonal Antibody G36-19 | - Li 2008, Cancer Epidemiology, Biomarkers & Prevention, Vol. 17:9, p. 2252 |
| | | | - Lundström 1994, Dermatological Research, Vol. 286, p. 369 |
| Interleukin-6 | Melanoma | IL-6 receptor | - Molnar 2006, Cancer Biology, Vol. 10, p. 25 |
| | | | - Angelis 1998, Neuroscience Letters, Vol. 244, p. 106-108 |
| S100 | Melanoma | RAGE | - Torabian 2005, Melanoma Biomarkers, Vol. 17, p. 167 ff |
| | | | - Donato 2001, Int. J. Biochem. & Cell Biol., Vol. 33, p. 637 ff |
| Dihydroxyindole (DHI) and derivates, such as 5,6-dihydroxyindole-2-carboxylic acid (DHIC2) | Melanoma | Anti-DHI antibodies | Slominski 2001, Arch Pathol Lab Med, Vol. 125, pp. 1295 ff. Kammeyer 1992, J Immunol Methods, Vol. 156, p. 61 ff |
| O-methyl derivates of DHI and DHICA | Melanoma | Anti-DHI antibodies | Slominski 2001, Arch Pathol Lab Med, Vol. 125, pp. 1295 ff |
| Tyrosinase | Melanoma | T-Cells in association with HLA-A2, HLA-A24, HLA-B44, HLA-A1, HLA-DR4, HLA-Dr15 | Slominski 2001, Arch Pathol Lab Med, Vol. 125, pp. 1295 ff |
| TRP-1, TRP-2 | Melanoma | T-Cells in association with HLA-A2, HLA-A24, HLA-B44, HLA-A1, HLA-DR4, HLA-Dr15 | Slominski 2001, Arch Pathol Lab Med, Vol. 125, pp. 1295 ff |
| HMB-45 | Melanoma | T-Cells in association with HLA-A2, HLA-A24, HLA-B44, HLA-A1, HLA-DR4, HLA-Dr15 | Slominski 2001, Arch Pathol Lab Med, Vol. 125, pp. 1295 ff |
| Malanocyte specific MART-1 | Melanoma | T-Cells in association with HLA-A2, HLA-A24, HLA-B44, HLA-A1, HLA-DR4, HLA-Dr15 | Slominski 2001, Arch Pathol Lab Med, Vol. 125, pp. 1295 ff |
| MAGE proteins | Melanoma | T-Cells in association with HLA-A2, HLA-A24, HLA-B44, HLA-A1, HLA-DR4, HLA-Dr15 | Slominski 2001, Arch Pathol Lab Med, Vol. 125, pp. 1295 ff |
| Styrene | Lung cancer | styrene dioxygenase, 1.14.12.12, styrene monooxygenase | Phillips et al. (1999), The Lancet, Vol. 353, pp. 1930-1935, KEGG database: www.genome.jp/kegg/ |
| Butane/cyclopropane | Lung cancer | GABA-A receptor | Krasowski and Harrison (2000), British Journal of Pharmacology, Vol. 129, pp. 731-743 |
| Styrene/methylpentane | Lung cancer | P450 CAM | Chen et al. (1999), Current Opinion in Biotechnology, Vol. 10, pp. 137-141 |
| Polycyclic aromatic hydrocarbons - in particular: naphthalene, fluorene, acenaphthene, acenaphthylene, 9-methylanthracene | All cancers | P450-BM3 | Li et al. (2001), Applied and Environmental Microbiology, December 2001, pp. 5735-5739 |
| Polycyclic or halogenated aromatic hydrocarbon ligands | All cancers | Aryl hydrocarbon receptor (PAS family) | Tzameli et al. (2000), Molecular and Cellular Biology, Vol. 20 (9), pp. 2951-2958 |
| n-Alkanes | Lung cancers | are1/yas1 (yeast) and cyp450alkl-8 | Yamagami et al. (2004), The Journal of Biological Chemistry, Vol. 279 (21), pp. 22183-22189 |
| Phenobarbital | All cancers | car | Tzameli et al. (2000), Molecular and Cellular Biology, Vol. 20 (9), pp. 2951-2958 |

**The figures show:**
- Fig. 1:: Growth curve of sender cells (A), AHL concentrations in the supernatant at different timepoints (B)
- Fig. 2:: Killing efficiency of colicinE1-receiver (the killer strain). The bars show the GFP-intensity of the prey cells at t=0 h and t=12 h for different prey-killer and prey-reference ratios

The following Examples are given to illustrate the invention. They shall not be construed as to limit the scope of the invention.

### Example 1: Construction of the prey strain

LuxS (SEQ ID NO. 3) was amplified from the *V*. *harveyi* genome and cloned into pTrc99α (SEQ ID NO. 5)with NcoI/BamHI. Restriction sites were introduced into the gene via the PCR primers. Subsequently the construct was cloned into *E. coli* DH5α. LuxS expression is controlled via an IPTG-inducible promoter.

### Example 2: Construction of the killer strain

First LuxQ and Tar parts for each Fusion receptor were amplified. LuxQ was taken from *V. harveyi* genome, Tar from pDK48 (SEQ ID NO. 6). Reverse Primers of LuxQ and forward primers of Tar were complementary, thus making it possible to fuse both parts together in a second PCR. Afterwards the Fusion constructs were cloned into pDK48 with NcoI/NdeI. AI-2 binding to LuxQ also requires LuxP (SEQ ID NO. 4) wherefore this was also amplified from *V. harveyi.* First it was cloned into native pDK48 with SalI/NotI and later on pDK48 containing the Fusion receptors at the same sites. It was necessary to first produce the Fusion constructs, because of conflicting restriction sites. Subsequently the constructs were transformed into *E. coli* MG1655 and HCB33 which could be used to the test the constructs in swarm assays. Since those two strains also contain other chemotaxis receptors swarm assays needed to be performed on minimal medium where cells do not grow so well. Therefore UU1250, a knock-out strain for chemotaxis receptors, was also used for swarm assay. Yet there was a conflict with antibiotic resistance. UU1250 have Kanamycin resistance encoded on the genome which is also on pDK48. Therefore the Fusion constructs were cloned into pBAD33 (SEQ ID NO. 7; Chloramphenicol resistance) with BamHI/PstI and then transformed into UU1250. Expression of the Fusion receptor is controllable via an arabinose-inducible promoter, both on pDK48 and pBAD33.

### Example 3: Testing of the AI-2 production of the prey strain (sender activity test)

The sender activity test was performed to measure the efficiency of the AHL production of the sender cells. Therefore TB-media with the appropriated antibiotic was inoculated from with 2 µl/ml of an overnight culture. For 7 hours every hour the OD of the medium was measured and 5 ml of the culture were spin down to produce supernatant. The sterile filtered supernatant, still containing the AHL, was stored at 4 °C. The supernatant of every hour was then added in different ratios to a constant amount of AHL inducible cells which produce GFP after induction. The OD and GFP intensities were measured at 37 °C in the Tecan Microplate Reader every half an hour for about 12 hours. The same test was carried out with amplifier cells. To calculate the produced amount of AHL by sender or amplifier cells as reference the test were performed also with different AHL concentration (0 M-100 nM) instead of AHL producing cells.

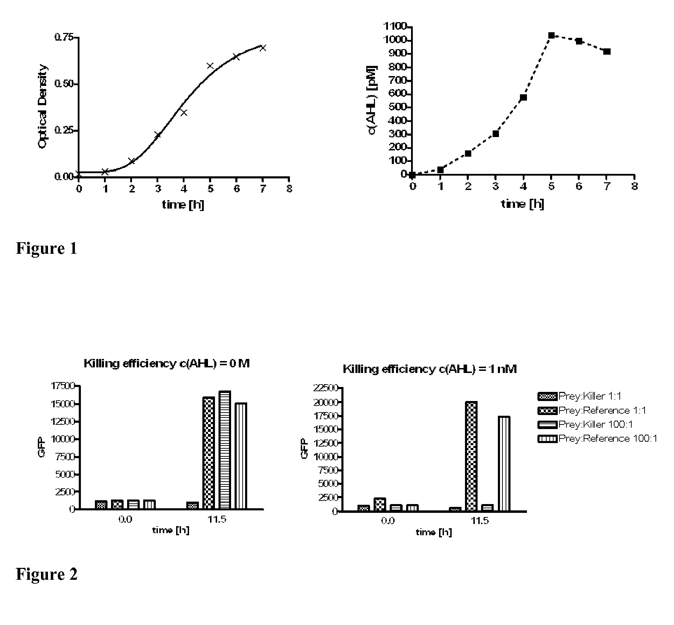

As shown in Fig. 1 the production of N-Acyl-Homoserin-Lactone (1b) increased in parallel with the cell density (1a) of the prey strain.

### Example 4: Testing of the killing module of the killer strain

To measure the killing efficiency and which amount of cells or colicins are needed to reach any killing activity a colicin activity test was carried out. Therefore bacteria containing the colicin plasmid (TOP10 or MG1655) and GFP producing cells (reference promoter, TOP10) were inoculated in TB-media with appropriated antibiotics at 37 °C for 4 to 6 hours and the optical density of the two strains was adapted. The colicin cells themselves, their produced supernatant or the supernatant of the lysed colicin cells were added in different ratios to a constant amount of GFP producing cells. The total volume was kept constant and the missing amount added with TB-media without antibiotics. The colicin production was induced by several concentrations (0 M-100 nM) of *N*-Acyl-Homoserin-Lactone (AHL). The OD and GFP intensities were measured at 37 °C in the Tecan Microplate Reader every half an hour for about 12 hours.

As a negative control the similar test was carried out with cells, containing the same plasmid without the colicin gene on it.

The two bar diagrams in Fig. 2 show the killing efficiency of colicinE1-receive in dependence on AHL concentrations and prey-killer ratios. In the case of a 1:1 ratio the killer cells kill all prey cells at an AHL concentration of 0 M and 1 nM. The leakiness of the P_{LuxR} promoter could be responsible for that. At a prey-killer ratio of 5:1 up to 100:1 there is an OFF-state for c(AHL) = 0 M and an ON-state for c(AHL) = 1 nM. In the OFF-state colicin production is to low to harm the prey population efficiently, but in the ON-state enough colicin E1 is produced and released to kill all the prey cells. For all tests a negative controls was performed by using the killer cells without the harmful colicin producing gene (reference cells). In these tests the prey cells grew, which confirm the killing effect of the colicin producing cells.

## Claims

**1.** A chemotaxis fusion-receptor comprising
a) a signal transduction component; and
b) a periplasmic domain comprising a ligand binding site for a ligand selected from the group of ligands given in table 1.

**2.** The chemotaxis fusion receptor of claim 1, wherein the signal transduction component is derived from the chemotaxis system tar.

**3.** The chemotaxis fusion-receptor of claim 1 or 2, wherein the ligand binding site is the ligand binding site of a receptor selected from the group of receptors given in table 1.

**4.** A polynucleotide comprising genes encoding the chemotaxis fusion-receptor of any one of claims 1 to 3.

**5.** A vector comprising the polynucleotide of claim 4.

**6.** A bacterial host cell comprising the chemotaxis fusion-receptor of any of claims 1 to 3, the polynucleotide of claim 4, or the vector of claim 5.

**7.** The bacterial host cell of claim 6, wherein the bacterium belongs to the genera *Clostridium, Escherichia*, *Salmonella* and *Bifidobacterium.*

**8.** The bacterial host cell of claim 6 or 7, wherein the host cell comprises at least two different chemotaxis fusion-receptors according to any of claims 1 to 3, wherein the different fusion-receptors are capable of binding different ligands.

**9.** The host cell of claim 6 to 8, further comprising a polynucleotide comprising at least one nucleic acid encoding a protein which enables the bacterial host cell to kill cancer cells.

**11.** The host cell of claim 9, wherein said protein is a colicin.

**12.** Use of the host cell of any one of claims 6 to 11 for the manufacture of a pharmaceutical composition for the treatment of cancer.

**13.** The use of claim 12, wherein the cancer is lung cancer, a melanoma or oral submucous fibrosis.

**14.** A method for treating cancer in a subject comprising the step of administering the host cell of any one of claims 6 to 11 to a subject suffering from cancer in a therapeutically active amount.


SEQUENCE LISTING

<110>  DKFZ Deutsches Krebsforschungszentrum, Stiftung des
       öffentlichen Rechts

<120>  Use of bacteria for the sensing and killing of cancer cells

<130>  DK66052EP

<160>  7

<170>  PatentIn version 3.5

<210>  1
<211>  564
<212>  PRT
<213>  Escherichia coli

<400>  1

Met Asp Glu Leu Tyr Lys Gly Gly Gly Gly Ser Val Ile Asn Arg Ile
1               5                   10                  15

Arg Val Val Thr Leu Leu Val Met Val Leu Gly Val Phe Ala Leu Leu
            20                  25                  30

Gln Leu Ile Ser Gly Ser Leu Phe Phe Ser Ser Leu His His Ser Gln
        35                  40                  45

Lys Ser Phe Val Val Ser Asn Gln Leu Arg Glu Gln Gln Gly Glu Leu
    50                  55                  60

Thr Ser Thr Trp Asp Leu Met Leu Gln Thr Arg Ile Asn Leu Ser Arg
65                  70                  75                  80

Ser Ala Val Arg Met Met Met Asp Ser Ser Asn Gln Gln Ser Asn Ala
                85                  90                  95

Lys Val Glu Leu Leu Asp Ser Ala Arg Lys Thr Leu Ala Gln Ala Ala
            100                 105                 110

Thr His Tyr Lys Lys Phe Lys Ser Met Ala Pro Leu Pro Glu Met Val
        115                 120                 125

Ala Thr Ser Arg Asn Ile Asp Glu Lys Tyr Lys Asn Tyr Tyr Thr Ala
    130                 135                 140

Leu Thr Glu Leu Ile Asp Tyr Leu Asp Tyr Gly Asn Thr Gly Ala Tyr
145                 150                 155                 160

Phe Ala Gln Pro Thr Gln Gly Met Gln Asn Ala Met Gly Glu Ala Phe
                165                 170                 175

Ala Gln Tyr Ala Leu Ser Ser Glu Lys Leu Tyr Arg Asp Ile Val Thr
            180                 185                 190

Asp Asn Ala Asp Asp Tyr Arg Phe Ala Gln Trp Gln Leu Ala Val Ile
        195                 200                 205

Ala Leu Val Val Val Leu Ile Leu Leu Val Ala Trp Tyr Gly Ile Arg
    210                 215                 220

Arg Met Leu Leu Thr Pro Leu Ala Lys Ile Ile Ala His Ile Arg Glu
225                 230                 235                 240

Ile Ala Gly Gly Asn Leu Ala Asn Thr Leu Thr Ile Asp Gly Arg Ser
                245                 250                 255

Glu Met Gly Asp Leu Ala Gln Ser Val Ser His Met Gln Arg Ser Leu
            260                 265                 270

Thr Asp Thr Val Thr His Val Arg Glu Gly Ser Asp Ala Ile Tyr Ala
        275                 280                 285

Gly Thr Arg Glu Ile Ala Ala Gly Asn Thr Asp Leu Ser Ser Arg Thr
    290                 295                 300

Glu Gln Gln Ala Ser Ala Leu Glu Glu Thr Ala Ala Ser Met Glu Gln
305                 310                 315                 320

Leu Thr Ala Thr Val Lys Gln Asn Ala Asp Asn Ala Arg Gln Ala Ser
                325                 330                 335

Gln Leu Ala Gln Ser Ala Ser Asp Thr Ala Gln His Gly Gly Lys Val
            340                 345                 350

Val Asp Gly Val Val Lys Thr Met His Glu Ile Ala Asp Ser Ser Lys
        355                 360                 365

Lys Ile Ala Asp Ile Ile Ser Val Ile Asp Gly Ile Ala Phe Gln Thr
    370                 375                 380

Asn Ile Leu Ala Leu Asn Ala Ala Val Glu Ala Ala Arg Ala Gly Glu
385                 390                 395                 400

Gln Gly Arg Gly Phe Ala Val Val Ala Gly Glu Val Arg Asn Leu Ala
                405                 410                 415

Ser Arg Ser Ala Gln Ala Ala Lys Glu Ile Lys Ala Leu Ile Glu Asp
            420                 425                 430

Ser Val Ser Arg Val Asp Thr Gly Ser Val Leu Val Glu Ser Ala Gly
        435                 440                 445

Glu Thr Met Asn Asn Ile Val Asn Ala Val Thr Arg Val Thr Asp Ile
    450                 455                 460

Met Gly Glu Ile Ala Ser Ala Ser Asp Glu Gln Ser Arg Gly Ile Asp
465                 470                 475                 480

Gln Val Ala Leu Ala Val Ser Glu Met Asp Arg Val Thr Gln Gln Asn
                485                 490                 495

Ala Ser Leu Val Gln Glu Ser Ala Ala Ala Ala Ala Ala Leu Glu Glu
            500                 505                 510

Gln Ala Ser Arg Leu Thr Gln Ala Val Ser Ala Phe Arg Leu Ala Ala
        515                 520                 525

Ser Pro Leu Thr Asn Lys Pro Gln Thr Pro Ser Arg Pro Ala Ser Glu
    530                 535                 540

Gln Pro Pro Ala Gln Pro Arg Leu Arg Ile Ala Glu Gln Asp Pro Asn
545                 550                 555                 560

Trp Glu Thr Phe

<210>  2
<211>  594
<212>  PRT
<213>  Vibrio harveyi

<400>  2

Met Asn Lys Phe Arg Leu Ala Asn Ala Ala Leu Phe Val Ile Ile Thr
1               5                   10                  15

Val Met Ala Val Leu Ala Cys Met Thr His Val Asn Ser Arg Asn Thr
            20                  25                  30

Gln Ala Val Tyr Thr Ser Leu Ser Glu Ile Gly His His Leu Ile Glu
        35                  40                  45

Ala Arg Asp Ser Val Val Asn Pro Tyr Ser Ile Arg Glu Arg Arg Asn
    50                  55                  60

Tyr Glu Ile Thr His Ser Leu Val Glu Leu Glu Leu Ala Ser Glu Gln
65                  70                  75                  80

Leu Ile Ser Asp Phe Lys Gln Ser Ile Trp Phe Asp Ile Thr Ser Thr
                85                  90                  95

Arg Leu Arg Ala Gln Ser Ile Val Val Gln Phe Asn Gly Lys Val Arg
            100                 105                 110

Asp Ala Thr Gln Ser Leu Asp Met Leu Ile Gly Val Gln Val Ala Asn
        115                 120                 125

Gln Tyr Glu Leu Leu Thr Leu His Asn Ile Tyr Lys Asp Gln Phe Ser
    130                 135                 140

Ala Gln Thr Asp Asp Ile Trp Leu Glu Glu His Tyr Phe Asp Phe Leu
145                 150                 155                 160

Thr Asn Ala Ala Leu Gln Gly Asp Asn Pro Gln Ser Gln Ser Ile Ser
                165                 170                 175

Leu Phe Leu Asn Arg Leu Arg Gln Ser Glu Arg Lys Ile Glu Leu Leu
            180                 185                 190

Thr Asn Arg Ile Leu Glu Gly His Tyr Phe Glu Phe Val Glu Tyr Ser
        195                 200                 205

Glu His Gln Leu Leu Glu Ile Ala Gln Arg Glu Ser Arg Leu Thr Trp
    210                 215                 220

Leu Phe Val Leu Leu Ser Ile Ile Leu Leu Val Thr Ala Phe Val Leu
225                 230                 235                 240

Gln Thr Gln His Arg Val Asn Lys Leu Lys His Leu Asn Asp Glu Leu
                245                 250                 255

Ile Glu Ala Thr Glu Lys Ala Glu Arg Ala Ala Lys Ala Lys Ser Gln
            260                 265                 270

Phe Leu Ala Thr Met Ser His Glu Leu Arg Thr Pro Met Asn Gly Val
        275                 280                 285

Leu Gly Ile Ser Gln Ile Ile Ala Asn Glu Thr Gln Glu Lys Thr Thr
    290                 295                 300

Lys Glu His Val Lys Ile Ile Leu Asp Ser Val Gln His Leu Met Thr
305                 310                 315                 320

Ile Leu Asn Asp Ile Leu Asp Phe Ser Lys Val Glu Glu Asn Arg Leu
                325                 330                 335

Asp Leu Glu Ala Ala Pro Phe Asn Leu Leu Gln Val Leu Thr Pro Val
            340                 345                 350

Cys Ser Ala Ile Gln Pro Leu Val Glu Glu Lys Asn Ile Gln Leu Tyr
        355                 360                 365

Val Glu Asn Glu Val Pro Asp Thr Ile Glu Phe Lys Gly Asp Cys Ala
    370                 375                 380

Arg Val Arg Gln Ile Leu Phe Asn Leu Ala Gly Asn Ala Val Lys Phe
385                 390                 395                 400

Thr Gly Asp Gly His Val Leu Ile Arg Ala Glu Leu Asp Glu Gln Lys
                405                 410                 415

Arg Cys Leu Leu Ile Ser Val Asn Asp Thr Gly Ile Gly Ile Pro Gly
            420                 425                 430

Asp Lys Gln Gly Cys Ile Phe Asn Ser Phe Glu Gln Ala Asp Thr Ser

435                 440                 445

Thr Thr Arg Lys Phe Gly Gly Thr Gly Leu Gly Leu Ala Ile Val Lys
    450                 455                 460

Lys Leu Thr Glu Leu Met Ser Gly Glu Ile Lys Leu Lys Ser Val Glu
465                 470                 475                 480

Ser Ile Gly Thr Gln Phe Val Val Glu Leu Pro Ile Pro Trp Ile Glu
                485                 490                 495

Lys Glu Leu Pro Ala Thr Gln Asn Thr Pro Val Lys Gln Gln Arg Glu
            500                 505                 510

Ser Lys Arg His Leu His Ile Leu Leu Ala Glu Asp Asn Arg Val Asn
        515                 520                 525

Ala Ile Val Ala Lys Gly Phe Cys Glu Lys Leu Gly His Thr Val Glu
    530                 535                 540

Ile Ala Glu Asn Gly Leu Ile Ala Thr Lys Lys Ala Gln Glu His Gln
545                 550                 555                 560

Tyr Asp Leu Ile Leu Met Ser Asp Arg Arg His Leu Leu Pro Asp Ala
                565                 570                 575

Ala Leu Leu Arg Thr Val Arg Ala Thr Phe Thr Ala Tyr Gly Ser Ser
            580                 585                 590

Leu His


<210>  3
<211>  480
<212>  DNA
<213>  Vibrio harveyi

<400>  3
atgaatgcac cagcggttcg tgtggctaaa acgatgcaaa ctccaaaagg agacaccatc      60

acggtattcg acctacgttt cactgctcca aacaaagaca tcctttctga gaaaggaatt     120

catacattag agcatttgta cgcaggcttt atgcgtaatc acctaaatgg tgatagcgtt     180

gagatcattg atatctcacc aatggggtgc cgtactggtt tctacatgag cttgattggt     240

acgccttcag agcagcaagt ggctgacgct tggattgccg cgatggaaga cgtactaaaa     300

gtagaaaacc aaaacaagat ccctgagttg aacgaatacc aatgtggtac agcagcgatg     360

cactctctgg atgaagcgaa gcaaatcgcg aagaacattc tagaagtggg tgtggcggtg     420

aataagaatg atgaattggc actgccagag tcaatgctga gagagctacg catcgactaa     480

<210>  4
<211>  1098
<212>  DNA
<213>  Vibrio harveyi

<400>  4
atgaagaaag cgttactatt ttccctgatt tctatggtcg gtttttctcc agcgtctcaa      60

gcaacacaag ttttgaatgg gtactggggt tatcaagagt ttttggacga gtttcccgag     120

caacgaaatc tgaccaatgc tttatcagaa gcagtacgag cacagccggt cccactgtct     180

aaaccgacac aacgcccgat taaaatatca gtggtttacc caggacagca agtttcagat     240

tactgggtaa gaaatattgc atcattcgaa aaacgtttgt ataagttgaa tattaactac     300

caactgaacc aagtgtttac tcgtccaaat gctgatatca agcaacaaag cttgtcatta     360

atggaagcgc tcaagagcaa atcggattac ttgattttca cgcttgatac gacaagacac     420

cgtaaatttg ttgagcacgt tttggactca acgaacacca aattgatctt gcaaaatatc     480

actacaccag tccgtgagtg ggacaaacat caaccgtttt tatatgtcgg atttgaccac     540

gcagaaggca gtcgtgaatt agcaacagaa ttcggaaagt tcttcccaaa acacacatat     600

tacagtgtgc tctacttttc tgaaggttat attagcgatg tgagaggtga tacttttatt     660

caccaagtaa accgtgataa taactttgag ctacaatcag cgtattacac gaaggcaacc     720

aagcaatccg gctatgatgc tgcgaaagcg agtttagcaa aacatccaga tgttgatttt     780

atctatgcat gttcgaccga cgtagcatta ggtgcagtag acgcactggc tgagttggga     840

cgtgaagata ttatgatcaa tggctggggt ggaggctctg ctgagttaga cgctatccag     900

aagggtgatt tagacatcac cgtcatgcgt atgaatgatg acactggcat agccatggca     960

gaagcgatta agtgggactt ggaagataaa ccagttccga ccgtatactc aggtgacttt    1020

gaaatcgtaa caaaggcaga ttcaccggag agaatcgaag cgctgaaaaa gcgcgcattt    1080

agatattcag ataattga                                                  1098

<210>  5
<211>  4176
<212>  DNA
<213>  Escherichia coli

<400>  5
gtttgacagc ttatcatcga ctgcacggtg caccaatgct tctggcgtca ggcagccatc      60

ggaagctgtg gtatggctgt gcaggtcgta aatcactgca taattcgtgt cgctcaaggc     120

gcactcccgt tctggataat gttttttgcg ccgacatcat aacggttctg gcaaatattc     180

tgaaatgagc tgttgacaat taatcatccg gctcgtataa tgtgtggaat tgtgagcgga     240

taacaatttc acacaggaaa cagaccatgg aattcgagct cggtacccgg ggatcctcta     300

gagtcgacct gcaggcatgc aagcttggct gttttggcgg atgagagaag attttcagcc     360

tgatacagat taaatcagaa cgcagaagcg gtctgataaa acagaatttg cctggcggca     420

gtagcgcggt ggtcccacct gaccccatgc cgaactcaga agtgaaacgc cgtagcgccg     480

atggtagtgt ggggtctccc catgcgagag tagggaactg ccaggcatca aataaaacga     540

aaggctcagt cgaaagactg ggcctttcgt tttatctgtt gtttgtcggt gaacgctctc     600

ctgagtagga caaatccgcc gggagcggat ttgaacgttg cgaagcaacg gcccggaggg     660

tggcgggcag gacgcccgcc ataaactgcc aggcatcaaa ttaagcagaa ggccatcctg     720

acggatggcc tttttgcgtt tctacaaact ctttttgttt atttttctaa atacattcaa 780
atatgtatcc gctcatgaga caataaccct gataaatgct tcaataatat tgaaaaagga 840
agagtatgag tattcaacat ttccgtgtcg cccttattcc cttttttgcg gcattttgcc 900
ttcctgtttt tgctcaccca gaaacgctgg tgaaagtaaa agatgctgaa gatcagttgg 960
gtgcacgagt gggttacatc gaactggatc tcaacagcgg taagatcctt gagagttttc 1020
gccccgaaga acgttttcca atgatgagca cttttaaagt tctgctatgt ggcgcggtat 1080
tatcccgtgt tgacgccggg caagagcaac tcggtcgccg catacactat tctcagaatg 1140
acttggttga gtactcacca gtcacagaaa agcatcttac ggatggcatg acagtaagag 1200
aattatgcag tgctgccata accatgagtg ataacactgc ggccaactta cttctgacaa 1260
cgatcggagg accgaaggag ctaaccgctt ttttgcacaa catgggggat catgtaactc 1320
gccttgatcg ttgggaaccg gagctgaatg aagccatacc aaacgacgag cgtgacacca 1380
cgatgcctac agcaatggca acaacgttgc gcaaactatt aactggcgaa ctacttactc 1440
tagcttcccg gcaacaatta atagactgga tggaggcgga taaagttgca ggaccacttc 1500
tgcgctcggc ccttccggct ggctggttta ttgctgataa atctggagcc ggtgagcgtg 1560
ggtctcgcgg tatcattgca gcactggggc cagatggtaa gccctcccgt atcgtagtta 1620
tctacacgac ggggagtcag gcaactatgg atgaacgaaa tagacagatc gctgagatag 1680
gtgcctcact gattaagcat tggtaactgt cagaccaagt ttactcatat atactttaga 1740
ttgatttaaa acttcatttt taatttaaaa ggatctaggt gaagatcctt tttgataatc 1800
tcatgaccaa aatcccttaa cgtgagtttt cgttccactg agcgtcagac cccgtagaaa 1860
agatcaaagg atcttcttga gatccttttt ttctgcgcgt aatctgctgc ttgcaaacaa 1920
aaaaaccacc gctaccagcg gtggtttgtt tgccggatca agagctacca actctttttc 1980
cgaaggtaac tggcttcagc agagcgcaga taccaaatac tgtccttcta gtgtagccgt 2040
agttaggcca ccacttcaag aactctgtag caccgcctac atacctcgct ctgctaatcc 2100
tgttaccagt ggctgctgcc agtggcgata agtcgtgtct taccgggttg gactcaagac 2160
gatagttacc ggataaggcg cagcggtcgg gctgaacggg gggttcgtgc acacagccca 2220
gcttggagcg aacgacctac accgaactga gatacctaca gcgtgagcta tgagaaagcg 2280
ccacgcttcc cgaagggaga aaggcggaca ggtatccggt aagcggcagg gtcggaacag 2340
gagagcgcac gagggagctt ccagggggaa acgcctggta tctttatagt cctgtcgggt 2400
ttcgccacct ctgacttgag cgtcgatttt tgtgatgctc gtcagggggg cggagcctat 2460
ggaaaaacgc cagcaacgcg gcctttttac ggttcctggc cttttgctgg ccttttgctc 2520
acatgttctt tcctgcgtta tcccctgatt ctgtggataa ccgtattacc gcctttgagt 2580
gagctgatac cgctcgccgc agccgaacga ccgagcgcag cgagtcagtg agcgaggaag 2640
cggaagagcg cctgatgcgg tattttctcc ttacgcatct gtgcggtatt tcacaccgca 2700
tatggtgcac tctcagtaca atctgctctg atgccgcata gttaagccag tatacactcc 2760
gctatcgcta cgtgactggg tcatggctgc gccccgacac ccgccaacac ccgctgacgc 2820

gccctgacgg gcttgtctgc tcccggcatc cgcttacaga caagctgtga ccgtctccgg 2880

gagctgcatg tgtcagaggt tttcaccgtc atcaccgaaa cgcgcgaggc agcagatcaa 2940

ttcgcgcgcg aaggcgaagc ggcatgcatt tacgttgaca ccatcgaatg gtgcaaaacc 3000

tttcgcggta tggcatgata gcgcccggaa gagagtcaat tcagggtggt gaatgtgaaa 3060

ccagtaacgt tatacgatgt cgcagagtat gccggtgtct cttatcagac cgtttcccgc 3120

gtggtgaacc aggccagcca cgtttctgcg aaaacgcggg aaaaagtgga agcggcgatg 3180

gcggagctga attacattcc caaccgcgtg gcacaacaac tggcgggcaa acagtcgttg 3240

ctgattggcg ttgccacctc cagtctggcc ctgcacgcgc cgtcgcaaat tgtcgcggcg 3300

attaaatctc gcgccgatca actgggtgcc agcgtggtgg tgtcgatggt agaacgaagc 3360

ggcgtcgaag cctgtaaagc ggcggtgcac aatcttctcg cgcaacgcgt cagtgggctg 3420

atcattaact atccgctgga tgaccaggat gccattgctg tggaagctgc ctgcactaat 3480

gttccggcgt tatttcttga tgtctctgac cagacaccca tcaacagtat tattttctcc 3540

catgaagacg gtacgcgact gggcgtggag catctggtcg cattgggtca ccagcaaatc 3600

gcgctgttag cgggcccatt aagttctgtc tcggcgcgtc tgcgtctggc tggctggcat 3660

aaatatctca ctcgcaatca aattcagccg atagcggaac gggaaggcga ctggagtgcc 3720

atgtccggtt ttcaacaaac catgcaaatg ctgaatgagg gcatcgttcc cactgcgatg 3780

ctggttgcca acgatcagat ggcgctgggc gcaatgcgcg ccattaccga gtccgggctg 3840

cgcgttggtg cggatatctc ggtagtggga tacgacgata ccgaagacag ctcatgttat 3900

atcccgccgt caaccaccat caaacaggat tttcgcctgc tggggcaaac cagcgtggac 3960

cgcttgctgc aactctctca gggccaggcg gtgaagggca atcagctgtt gcccgtctca 4020

ctggtgaaaa gaaaaaccac cctggcgccc aatacgcaaa ccgcctctcc ccgcgcgttg 4080

gccgattcat taatgcagct ggcacgacag gtttcccgac tggaaagcgg gcagtgagcg 4140

caacgcaatt aatgtgagtt agcgcgaatt gatctg 4176

<210> 6
<211> 6601
<212> DNA
<213> Escherichia coli

<400> 6
atcgatgcat aatgtgcctg tcaaatggac gaagcaggga ttctgcaaac cctatgctac 60

tccgtcaagc cgtcaattgt ctgattcgtt accaattatg acaacttgac ggctacatca 120

ttcacttttt cttcacaacc ggcacggaac tcgctcgggc tggccccggt gcatttttta 180

aatacccgcg agaaatagag ttgatcgtca aaaccaacat tgcgaccgac ggtggcgata 240

ggcatccggg tggtgctcaa aagcagcttc gcctggctga tacgttggtc ctcgcgccag 300

cttaagacgc taatccctaa ctgctggcgg aaaagatgtg acagacgcga cggcgacaag 360

caaacatgct gtgcgacgct ggcgatatca aaattgctgt ctgccaggtg atcgctgatg 420

tactgacaag cctcgcgtac ccgattatcc atcggtggat ggagcgactc gttaatcgct 480

tccatgcgcc gcagtaacaa ttgctcaagc agatttatcg ccagcagctc cgaatagcgc 540
ccttcccctt gcccggcgtt aatgatttgc ccaaacaggt cgctgaaatg cggctggtgc 600
gcttcatccg ggcgaaagaa ccccgtattg gcaaatattg acggccagtt aagccattca 660
tgccagtagg cgcgcggacg aaagtaaacc cactggtgat accattcgcg agcctccgga 720
tgacgaccgt agtgatgaat ctctcctggc gggaacagca aaatatcacc cggtcggcaa 780
acaaattctc gtccctgatt tttcaccacc ccctgaccgc gaatggtgag attgagaata 840
taacctttca ttcccagcgg tcggtcgata aaaaaatcga gataaccgtt ggcctcaatc 900
ggcgttaaac ccgccaccag atgggcatta aacgagtatc ccggcagcag gggatcattt 960
tgcgcttcag ccatactttt catactcccg ccattcagag aagaaaccaa ttgtccatat 1020
tgcatcagac attgccgtca ctgcgtcttt tactggctct tctcgctaac caaaccggta 1080
accccgctta ttaaaagcat tctgtaacaa agcgggacca aagccatgac aaaaacgcgt 1140
aacaaaagtg tctataatca cggcagaaaa gtccacattg attatttgca cggcgtcaca 1200
ctttgctatg ccatagcatt tttatccata agattagcgg atcctacctg acgcttttta 1260
tcgcaactct ctactgtttc tccatacccg tttttttggg ctagcaggag gaattcacca 1320
tggaattcga gctcaggagt gtgaaatggt gagcaagggc gaggagctgt tcaccggggt 1380
ggtgcccatc ctggtcgagc tggacggcga cgtaaacggc cacaagttca gcgtgtccgg 1440
cgagggcgag ggcgatgcca cctacggcaa gctgaccctg aagttcatct gcaccaccgg 1500
caagctgccc gtgccctggc ccaccctcgt gaccaccttc ggctacggcc tgcagtgctt 1560
cgcccgctac cccgaccaca tgaagcagca cgacttcttc aagtccgcca tgcccgaagg 1620
ctacgtccag gagcgcacca tcttcttcaa ggacgacggc aactacaaga cccgcgccga 1680
ggtgaagttc gagggcgaca ccctggtgaa ccgcatcgag ctgaagggca tcgacttcaa 1740
ggaggacggc aacatcctgg ggcacaagct ggagtacaac tacaacagcc acaacgtcta 1800
tatcatggcc gacaagcaga agaacggcat caaggtgaac ttcaagatcc gccacaacat 1860
cgaggacggc agcgtgcagc tcgccgacca ctaccagcag aacaccccca tcggcgacgg 1920
ccccgtgctg ctgcccgaca accactacct gagctaccag tccaagctga gcaaagaccc 1980
caacgagaag cgcgatcaca tggtcctgct ggagttcgtg accgccgccg ggatcactct 2040
cggcatggac gagctgtaca agggaggtgg aggatccgtg attaaccgta tccgcgtagt 2100
cacgctgttg gtaatggtgc tgggggtatt cgcactgtta cagcttattt ccggcagtct 2160
gtttttttct tcccttcacc atagccagaa gagctttgtg gtttccaatc aattacggga 2220
acagcagggc gagctgacgt caacctggga tttaatgctg caaacgcgca ttaacctgag 2280
tcgttcagcg gtacggatga tgatggattc ctccaatcaa caaagtaacg ccaaagttga 2340
attgctcgat agcgccagga aaacattggc gcaggcagcg acgcattata aaaaattcaa 2400
aagcatggca ccgttacctg aaatggtcgc taccagtcgt aatattgatg aaaaatataa 2460
aaactattac acagcgttaa ctgaactgat tgattatcta gattatggca atactggagc 2520
ttatttcgct cagccaaccc agggaatgca aaatgcaatg ggcgaagcgt ttgctcagta 2580

cgccctcagc agtgaaaaac tgtatcgcga tatcgtcact gacaacgcag atgattaccg 2640
atttgcccag tggcaactgg cggttatcgc gctggtggtg gtattgattc tgctggtggc 2700
gtggtacggc attcgccgta tgttgcttac tccgctggca aaaattattg ctcacattcg 2760
cgaaatcgcc ggtggtaacc tggcgaatac cctgaccatt gacgggcgca gtgaaatggg 2820
cgacctggcg cagagcgttt cacatatgca acgctctttg actgacaccg tcactcatgt 2880
ccgcgaaggt tcagatgcca tctatgccgg tacccgtgaa attgcggcgg gcaacaccga 2940
tctttcctcc cgtactgaac agcaggcatc cgcgctggaa gaaactgccg ccagcatgga 3000
gcagctcacc gcgacagtga agcaaaacgc cgataacgcc cgccaggcct cgcaactggc 3060
gcaaagtgcc tccgacaccg cccagcacgg cggcaaagtg gtggatggcg tagtgaaaac 3120
gatgcatgag atcgccgata gttcgaagaa aattgccgac attatcagcg ttatcgacgg 3180
tattgccttc cagactaata tcctcgcgct gaatgccgcg gttgaagccg cgcgtgcggg 3240
tgaacagggc cgtggttttg ccgtggtggc gggtgaagtg cgtaatcttg ccagtcgcag 3300
cgcccaggcg gcaaaagaga tcaaagccct cattgaagac tccgtctcac gcgttgatac 3360
cggttcggtg ctggtcgaaa gcgccgggga aacaatgaac aatatcgtca atgctgtcac 3420
tcgcgtgact gacattatgg gcgagattgc atcggcatcg gatgaacaga gccgtggcat 3480
cgatcaagtc gcattggcgg tttcggaaat ggatcgcgtc acgcaacaga acgcatcgct 3540
ggtgcaggaa tcagctgccg ccgccgctgc gctggaagaa caggcgagtc gtttaacgca 3600
agcagtttcc gcgttccgtc tggcagccag cccactcacc aataaaccgc aaacaccatc 3660
ccgtcctgcc agtgagcaac caccggctca gccacgactg cgaattgctg aacaagatcc 3720
aaactgggaa acattttgag cggccgcgac tctagagtcg acctgcaggc atgcaagctt 3780
ggctgttttg gcggatgaga gaagattttc agcctgatac agattaaatc agaacgcaga 3840
agcggtctga taaaacagaa tttgcctggc ggcagtagcg cggtggtccc acctgacccc 3900
atgccgaact cagaagtgaa acgccgtagc gccgatggta gtgtggggtc tccccatgcg 3960
agagtaggga actgccaggc atcaaataaa acgaaaggct cagtcgaaag actgggcctt 4020
tcgttttatc tgttgtttgt cggtgaacgc tctcctgagt aggacaaatc cgccgggagc 4080
ggatttgaac gttgcgaagc aacggcccgg agggtggcgg gcaggacgcc cgccataaac 4140
tgccaggcat caaattaagc agaaggccat cctgacggat ggcctttttg cgtttctaca 4200
aactcttttg tttatttttc taaatacatt caaatatgta tccgctcatg agacaataac 4260
cctgataaat gcttcaataa tattgaaaaa ggaagagtat gagtattcaa catttccgtg 4320
tcgcccttat tccctttttt gcggcatttt gccttcctgt ttttgctcac ccagaaacgc 4380
tggtgaaagt aaaagatgct gaagatcagt tgggtgcacg agtgggttac atcgaactgg 4440
atctcaacag cggtaagatc cttgagagtt ttcgccccga agaacgtttt ccaatgatga 4500
gcacttttaa agttctgcta tgtggcgcgg tattatcccg tgttgacgcc gggcaagagc 4560
aactcggtcg ccgcatacac tattctcaga atgacttggt tgagtggggg gggggggaaa 4620
gccacgttgt gtctcaaaat ctctgatgtt acattgcaca agataaaaat atatcatcat 4680

gaacaataaa actgtctgct tacataaaca gtaatacaag gggtgttatg agccatattc 4740

aacgggaaac gtcttgctcg aggccgcgat taaattccaa catggatgct gatttatatg 4800

ggtataaatg ggctcgcgat aatgtcgggc aatcaggtgc gacaatctat cgattgtatg 4860

ggaagcccga tgcgccagag ttgtttctga aacatggcaa aggtagcgtt gccaatgatg 4920

ttacagatga gatggtcaga ctaaactggc tgacggaatt tatgcctctt ccgaccatca 4980

agcattttat ccgtactcct gatgatgcat ggttactcac cactgcgatc cccgggaaaa 5040

cagcattcca ggtattagaa gaatatcctg attcaggtga aaatattgtt gatgcgctgg 5100

cagtgttcct gcgccggttg cattcgattc ctgtttgtaa ttgtcctttt aacagcgatc 5160

gcgtatttcg tctcgctcag gcgcaatcac gaatgaataa cggtttggtt gatgcgagtg 5220

attttgatga cgagcgtaat ggctggcctg ttgaacaagt ctggaaagaa atgcataagc 5280

ttttgccatt ctcaccggat tcagtcgtca ctcatggtga tttctcactt gataacctta 5340

tttttgacga ggggaaatta ataggttgta ttgatgttgg acgagtcgga atcgcagacc 5400

gataccagga tcttgccatc ctatggaact gcctcggtga gttttctcct tcattacaga 5460

aacggctttt tcaaaaatat ggtattgata atcctgatat gaataaattg cagtttcatt 5520

tgatgctcga tgagtttttc taagatatct ccctcctgtt cagctactga cggggtggtg 5580

cgtaacggca aaagcaccgc cggacatcag cgctagcgga gtgtatactg gcttactatg 5640

ttggcactga tgagggtgtc agtgaagtgc ttcatgtggc aggagaaaaa aggctgcacc 5700

ggtgcgtcag cagaatatgt gatacaggat atattccgct tcctcgctca ctgactcgct 5760

acgctcggtc gttcgactgc ggcgagcgga aatggcttac gaacggggcg gagatttcct 5820

ggaagatgcc aggaagatac ttaacaggga agtgagaggg ccgcggcaaa gccgtttttc 5880

cataggctcc gcccccctga caagcatcac gaaatctgac gctcaaatca gtggtggcga 5940

aacccgacag gactataaag ataccaggcg tttccccctg gcggctccct cgtgcgctct 6000

cctgttcctg cctttcggtt taccggtgtc attccgctgt tatggccgcg tttgtctcat 6060

tccacgcctg acactcagtt ccgggtaggc agttcgctcc aagctggact gtatgcacga 6120

accccccgtt cagtccgacc gctgcgcctt atccggtaac tatcgtcttg agtccaaccc 6180

ggaaagacat gcaaaagcac cactggcagc agccactggt aattgattta gaggagttag 6240

tcttgaagtc atgcgccggt taaggctaaa ctgaaaggac aagttttggt gactgcgctc 6300

ctccaagcca gttacctcgg ttcaaagagt tggtagctca gagaaccttc gaaaaaccgc 6360

cctgcaaggc ggttttttcg ttttcagagc aagagattac gcgcagacca aaacgatctc 6420

aagaagatca tcttattaat cagataaaat atttctaggc tcatgagccc gaagtggcga 6480

gcccgatctt ccccatcggt gatgtcggcg atataggcgc cagcaaccgc acctgtggcg 6540

ccggtgatgc cggccacgat gcgtccggcg tagaggatct gctcatgttt gacagcttat 6600

c 6601

<210> 7
<211> 5360
<212> DNA

<213>  Escherichia coli

<400>  7
atcgatgcat aatgtgcctg tcaaatggac gaagcaggga ttctgcaaac cctatgctac      60

tccgtcaagc cgtcaattgt ctgattcgtt accaattatg acaacttgac ggctacatca     120

ttcacttttt cttcacaacc ggcacggaac tcgctcgggc tggccccggt gcatttttta     180

aatacccgcg agaaatagag ttgatcgtca aaaccaacat tgcgaccgac ggtggcgata     240

ggcatccggg tggtgctcaa aagcagcttc gcctggctga tacgttggtc ctcgcgccag     300

cttaagacgc taatccctaa ctgctggcgg aaaagatgtg acagacgcga cggcgacaag     360

caaacatgct gtgcgacgct ggcgatatca aaattgctgt ctgccaggtg atcgctgatg     420

tactgacaag cctcgcgtac ccgattatcc atcggtggat ggagcgactc gttaatcgct     480

tccatgcgcc gcagtaacaa ttgctcaagc agatttatcg ccagcagctc cgaatagcgc     540

ccttcccctt gcccggcgtt aatgatttgc ccaaacaggt cgctgaaatg cggctggtgc     600

gcttcatccg ggcgaaagaa ccccgtattg gcaaatattg acggccagtt aagccattca     660

tgccagtagg cgcgcggacg aaagtaaacc cactggtgat accattcgcg agcctccgga     720

tgacgaccgt agtgatgaat ctctcctggc gggaacagca aaatatcacc cggtcggcaa     780

acaaattctc gtccctgatt tttcaccacc ccctgaccgc gaatggtgag attgagaata     840

taacctttca ttcccagcgg tcggtcgata aaaaaatcga gataaccgtt ggcctcaatc     900

ggcgttaaac ccgccaccag atgggcatta aacgagtatc ccggcagcag gggatcattt     960

tgcgcttcag ccatactttt catactcccg ccattcagag aagaaaccaa ttgtccatat    1020

tgcatcagac attgccgtca ctgcgtcttt tactggctct tctcgctaac caaaccggta    1080

accccgctta ttaaaagcat tctgtaacaa agcgggacca aagccatgac aaaaacgcgt    1140

aacaaaagtg tctataatca cggcagaaaa gtccacattg attatttgca cggcgtcaca    1200

ctttgctatg ccatagcatt tttatccata agattagcgg atcctacctg acgcttttta    1260

tcgcaactct ctactgtttc tccatacccg tttttttggg ctagcgaatt cgagctcggt    1320

acccggggat cctctagagt cgacctgcag gcatgcaagc ttggctgttt tggcggatga    1380

gagaagattt tcagcctgat acagattaaa tcagaacgca gaagcggtct gataaaacag    1440

aatttgcctg gcggcagtag cgcggtggtc ccacctgacc ccatgccgaa ctcagaagtg    1500

aaacgccgta gcgccgatgg tagtgtgggg tctccccatg cgagagtagg gaactgccag    1560

gcatcaaata aaacgaaagg ctcagtcgaa agactgggcc tttcgtttta tctgttgttt    1620

gtcggtgaac gctctcctga gtaggacaaa tccgccggga gcggatttga acgttgcgaa    1680

gcaacggccc ggagggtggc gggcaggacg cccgccataa actgccaggc atcaaattaa    1740

gcagaaggcc atcctgacgg atggcctttt tgcgtttcta caaactcttt tgtttatttt    1800

tctaaataca ttcaaatatg tatccgctca tgagacaata accctgataa atgcttcaat    1860

aatattgaaa aaggaagagt atgagtattc aacatttccg tgtcgccctt attccctttt    1920

ttgcggcatt ttgccttcct gtttttgctc acccagaaac gctggtgaaa gtaaaagatg    1980

ctgaagatca gttgggtgca gcaaactatt aactggcgaa ctacttactc tagcttcccg    2040

gcaacaatta atagactgga tggaggcgga taaagttgca ggaccacttc tgcgctcggc 2100
ccttccggct ggctggttta ttgctgataa atctggagcc ggtgagcgtg ggtctcgcgg 2160
tatcattgca gcactggggc cagatggtaa gccctcccgt atcgtagtta tctacacgac 2220
ggggagtcag gcaactatgg atgaacgaaa tagacagatc gctgagatag gtgcctcact 2280
gattaagcat tggtaactgt cagaccaagt ttactcatat atactttaga ttgatttacg 2340
cgccctgtag cggcgcatta agcgcggcgg gtgtggtggt tacgcgcagc gtgaccgcta 2400
cacttgccag cgccctagcg cccgctcctt tcgctttctt cccttccttt ctcgccacgt 2460
tcgccggctt tccccgtcaa gctctaaatc gggggctccc tttagggttc cgatttagtg 2520
ctttacggca cctcgacccc aaaaaacttg atttgggtga tggttcacgt agtgggccat 2580
cgccctgata gacggttttt cgccctttga cgttggagtc cacgttcttt aatagtggac 2640
tcttgttcca aacttgaaca acactcaacc ctatctcggg ctattctttt gatttataag 2700
ggattttgcc gatttcggcc tattggttaa aaaatgagct gatttaacaa aaatttaacg 2760
cgaattttaa caaaatatta acgtttacaa tttaaaagga tctaggtgaa gatccttttt 2820
gataatctca tgaccaaaat cccttaacgt gagttttcgt tccactgagc gtcagacccc 2880
gtagaaaaga tcaaaggatc ttcttgagat cctttttttc tgcgcgtaat ctgctgcttg 2940
caaacaaaaa aaccaccgct accagcggtg gtttgtttgc cggatcaaga gctaccaact 3000
ctttttccga aggtaactgg cttcagcaga gcgcagatac caaatactgt ccttctagtg 3060
tagccgtagt taggccacca cttcaagaac tctgtagcac cgcctacata cctcgctctg 3120
ctaatcctgt taccagtcag gcatttgaga agcacacggt cacactgctt ccggtagtca 3180
ataaaccggt aaaccagcaa tagacataag cggctattta acgaccctgc cctgaaccga 3240
cgaccgggtc gaatttgctt tcgaatttct gccattcatc cgcttattat cacttattca 3300
ggcgtagcac caggcgttta agggcaccaa taactgcctt aaaaaaatta cgccccgccc 3360
tgccactcat cgcagtactg ttgtaattca ttaagcattc tgccgacatg gaagccatca 3420
cagacggcat gatgaacctg aatcgccagc ggcatcagca ccttgtcgcc ttgcgtataa 3480
tatttgccca tggtgaaaac gggggcgaag aagttgtcca tattggccac gtttaaatca 3540
aaactggtga aactcaccca gggattggct gagacgaaaa acatattctc aataaaccct 3600
ttagggaaat aggccaggtt ttcaccgtaa cacgccacat cttgcgaata tatgtgtaga 3660
aactgccgga aatcgtcgtg gtattcactc cagagcgatg aaaacgtttc agtttgctca 3720
tggaaaacgg tgtaacaagg gtgaacacta tcccatatca ccagctcacc gtctttcatt 3780
gccatacgga attccggatg agcattcatc aggcgggcaa gaatgtgaat aaaggccgga 3840
taaaacttgt gcttattttt ctttacggtc tttaaaaagg ccgtaatatc cagctgaacg 3900
gtctggttat aggtacattg agcaactgac tgaaatgcct caaaatgttc tttacgatgc 3960
cattgggata tatcaacggt ggtatatcca gtgatttttt tctccatttt agcttcctta 4020
gctcctgaaa atctcgataa ctcaaaaaat acgcccggta gtgatcttat ttcattatgg 4080
tgaaagttgg aacctcttac gtgccgatca acgtctcatt ttcgccaaaa gttggcccag 4140

ggcttcccgg tatcaacagg gacaccagga tttatttatt ctgcgaagtg atcttccgtc 4200
acaggtattt attcggcgca aagtgcgtcg ggtgatgctg ccaacttact gatttagtgt 4260
atgatggtgt ttttgaggtg ctccagtggc ttctgtttct atcagctgtc cctcctgttc 4320
agctactgac ggggtggtgc gtaacggcaa aagcaccgcc ggacatcagc gctagcggag 4380
tgtatactgg cttactatgt tggcactgat gagggtgtca gtgaagtgct tcatgtggca 4440
ggagaaaaaa ggctgcaccg gtgcgtcagc agaatatgtg atacaggata tattccgctt 4500
cctcgctcac tgactcgcta cgctcggtcg ttcgactgcg gcgagcggaa atggcttacg 4560
aacggggcgg agatttcctg gaagatgcca ggaagatact taacagggaa gtgagagggc 4620
cgcggcaaag ccgtttttcc ataggctccg cccccctgac aagcatcacg aaatctgacg 4680
ctcaaatcag tggtggcgaa acccgacagg actataaaga taccaggcgt ttccccctgg 4740
cggctccctc gtgcgctctc ctgttcctgc ctttcggttt accggtgtca ttccgctgtt 4800
atggccgcgt ttgtctcatt ccacgcctga cactcagttc cgggtaggca gttcgctcca 4860
agctggactg tatgcacgaa ccccccgttc agtccgaccg ctgcgcctta tccggtaact 4920
atcgtcttga gtccaacccg gaaagacatg caaaagcacc actggcagca gccactggta 4980
attgatttag aggagttagt cttgaagtca tgcgccggtt aaggctaaac tgaaaggaca 5040
agttttggtg actgcgctcc tccaagccag ttacctcggt tcaaagagtt ggtagctcag 5100
agaaccttcg aaaaaccgcc ctgcaaggcg gttttttcgt tttcagagca agagattacg 5160
cgcagaccaa aacgatctca agaagatcat cttattaatc agataaaata tttctaggct 5220
catgagcccg aagtggcgag cccgatcttc cccatcggtg atgtcggcga tataggcgcc 5280
agcaaccgca cctgtggcgc cggtgatgcc ggccacgatg cgtccggcgt agaggatctg 5340
ctcatgtttg acagcttatc 5360